(19) Europäisches Patentamt
European Patent Office
Office européen des brevets

(11) Publication number: 0 300 675
A2

# (12) EUROPEAN PATENT APPLICATION

(21) Application number: 88306391.9

(22) Date of filing: 13.07.88

(51) Int. Cl.4: A61K 37/02 , A61K 31/19

(30) Priority: 21.07.87 CA 542684

(43) Date of publication of application:
25.01.89 Bulletin 89/04

(84) Designated Contracting States:
CH DE FR GB IT LI NL

(71) Applicant: MERCK FROSST CANADA INC.
16711 Trans-Canada Highway
Kirkland Quebec(CA)

(72) Inventor: Ford-Hutchinson, Anthony W.
69 Hyde Park
Beaconsfield Quebec H9W 5L7(CA)

(74) Representative: Hesketh, Alan, Dr.
European Patent Department Merck & Co.,
Inc. Terlings Park Eastwick Road
Harlow Essex, CM20 2OR(GB)

## (54) Method for the improvement of cyclosporine therapy.

(57) The present invention relates to a method for improving cyclosporine therapy, in particular a method for limiting cyclosporine induced nephrotoxicity which comprises the adjunct administration in a mammal of an effective amount of cyclosporine and an effective amount of an antithromboxane $A_2$ agent.

EP 0 300 675 A2

# METHOD FOR THE IMPROVEMENT OF CYCLOSPORINE THERAPY

## BACKGROUND OF THE INVENTION

The present invention relates to a method for improving cyclosporine therapy, in particular a method for limiting cyclosporine induced nephrotoxicity which comprises adjunct administration in a mammal of an effective amount of cyclosporine and an effective amount of an antithromboxane agent.

The cyclosporines comprise a class of structurally distinct, cyclic, poly-N-methylated undecapeptides having valuable pharmacological, in particular immunosuppressive, anti-inflammatory and anti-protozoal activity. The first of the cyclosporines to be isolated and the "parent" compound of the class, is the naturally occurring fungal metabolite known as cyclosporine A, the production and properties of which are described e.g. in U.S. Patent 4,117,118. Since the original discovery of cyclosporine A, a wide variety of naturally occurring cyclosporines have been isolated and identified and many further non-natural cyclosporines have been prepared by synthetic or semi-synthetic means or by the application of modified culture techniques. The class comprised by the cyclosporines is thus now substantial and includes, for example, the naturally occurring cyclosporines ($Thr^2$)-, ($Val^2$)- and ($Nva^2$)-cyclosporine (also known as cyclosporines C, D and G, respectively), as well as various semi-synthetic derivatives thereof, such as their dihydro derivatives (e.g. as disclosed in U.S. Patents 4,108,985; 4,210,581 and 4,220,641) including e.g. (Dihydro-$MeBmt^1$)-($Val^2$)-cyclosporine (also known as dihydrocyclosporine D) and other natural and artificial cyclosporines such as those disclosed in European Patent Publication no. 0,058,134 B1, for example [(D)-$Ser^3$]-cyclosporine.

In accordance with now conventional nomenclature for the cyclosporines, these are defined herein by reference to the structure of cyclosporine A. This is done by first indicating those residues in the molecule which differ from those present in cyclosporine A and then applying the term "cyclosporine" to characterize the remaining residues which are identical to those present in cyclosporine A. Cyclosporine A has the formula I:

```
┌A-B-Sar-MeLeu-Val-MeLeu-Ala-(D)Ala-MeLeu-MeLeu-MeVal┐
│1 2  3    4    5    6    7     8     9     10    11  │   (I)
└─────────────────────────────────────────────────────┘
```

wherein A represents the -MeBmt- [N-methyl-(4R)-4-but-2E-en-1-yl-4-methyl-(L)threonyl] residue of formula II

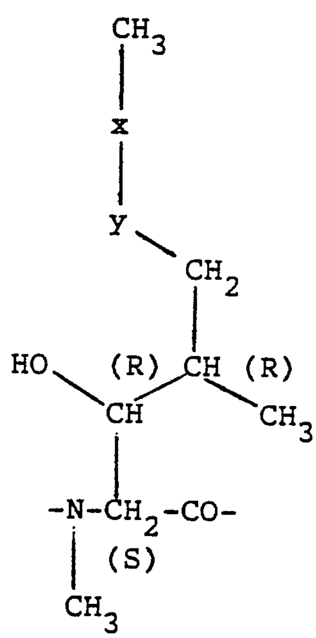

(II)

in which -x-y- is -CH=CH- (trans), and B is -α-Abu-. Accordingly, ($Thr^2$)-cyclosporine (cyclosporine C) is the compound of formula I, wherein A has the meaning given above and B is -Thr-, and (Dihydro-MeBmt')-($Val^2$)-cyclosporine (dihydrocyclosporine D) is the compound of formula I, wherein A represents the -dihydro-MeBmt- residue of formula II above in which -x-y- is $-CH_2-CH_2-$, and B is -Val-.

As the "parent" compound of the class, cyclosporine A has so far received the most attention. The primary area of clinical investigation for cyclosporine A has been as an immunosuppressive agent, in particular in relation to its application to recipients of organ transplants, e.g. heart, lung, combined heart-lung, liver, kidney, spleen, bone marrow, skin and corneal transplants. In this field cyclosporine A has achieved a remarkable success and reputation and is now commercially available and widely employed in clinic.

At the same time, applicability of cyclosporine A to various diseases thought to have an auto-immune basis (including for example: multiple sclerosis, Guillan -Barre syndrome, uveitis, myasthenia gravis, Heymann nephritis, juvenile diabetes type I, systemic lupus erythematosus, aplastic anaemia, pure red cell anaemia, idiopathic thrombocytopaenia, polychondritis, sclerodoma, Wegener granulamatosis, dermatomyositis, chronic active hepatitis, auto-immune male infertility, psoriasis and psoriatic arthritis, Steven-Johnson syndrome, idiopathic sprue, Chrone's disease, sarcoidosis, glomerulonephritis, interstitial lung fibrosis and primary billiary cirrhosis) and to inflammatory conditions, in particular inflammatory conditions with an aetiology including an auto-immune component such as arthritis and rheumatic diseases, has been intensive and reports and results in vitro, in animal models and in clinical trials are wide-spread in the literature.

A further area of investigation of cyclosporines has been the potential applicability as an anti-parasitic, in particular anti-protozoal agent, with possible uses suggested including malaria treatment and treatment of schistosomiasis, filariasis, leishmania and coccidiomycosis.

Other cyclosporines exhibit the same overall pharmacological utility as cyclosporine A and various proposals for application, in particular in one or other of the above identified indications, are prevelant in the literature.

Despite the very major contribution which cyclosporine A has made to the art, in particular in the field of transplant surgery, and despite cyclosporine A's wide acceptance and success in the clinic, an obvious negative feature has been its reported nephrotoxicity. Thus, means of reducing this side effect would clearly be of major benefit.

In accordance with the present invention, it has now been found that cyclosporine-induced nephrotoxicity in particular cyclosporine A, can be limited by adjunct administration of an antithromboxane agent.

## SUMMARY OF THE INVENTION

The invention provides a method of limiting cyclosporine-induced nephrotopicity which comprises the adjunct administration in a mammal of an effective amount of cyclosporine and an effective amount of an antithromboxane $A_2$ agent.

## DETAILED DESCRIPTION OF THE INVENTION

The invention provides a method for limiting cyclosporine-induced nephrotoxicity which comprises the adjunct administration in a mammal of an effective amount of cyclosporine and an effective amount of an antithromboxane $A_2$ agent.

The method of the invention can be utilized with any use of cyclosporine therapy. For example, the method can be utilized in all of the uses described hereinabove, with organ transplants being the most prevalent use.

The preferred cyclosporine of the invention is cyclosporine A.

As previously indicated, the method of the invention is particularly applicable to limiting cyclosporine-induced nephrotoxicity. Kidney damage, e.g. functional changes such as appearance of proteinuria, changes in renal blood flow and glomerular filtration rate and changes in electrolyte excretion and inulin clearance.

In accordance with the method of the present invention, cyclosporine and an antithromboxane $A_2$ agent can be administered separately at different times during the course of therapy or substantially concommitantly. Thus treatment with the antithromboxane $A_2$ agent can commence prior to cyclosporine treatment, or subsequent to commencement of cyclosporine treatment. The present invention is to be understood as embracing all such regimes of treatment and the term "adjunct administration" is to be interpreted

accordingly.

Doses of cyclosporine to be administered in practicing the method of the invention will of course vary depending upon, e.g. the particular cyclosporine employed, the mode of administration, the condition to be treated (e.g. whether treatment is for the purposes of immunosuppression or otherwise, and if for immunosuppression whether for use in relation to e.g. organ transplant, bone marrow transplant, or the treatment of auto-immune disease), as well as the effect desired. In addition, dosaging will generally require adjustment for individual patients in order to establish an appropriate long term drug serum concentration, e.g. by administration of an initial daily starting or "loading" dose with subsequent dose adjustment (generally dose reduction) in accordance with serum levels, e.g. as determined by regular radioimmunoassay (RIA) monitoring.

In general, amounts administered will be of the same order to those conventionally employed in cyclosporine therapy, e.g. cyclosporine A therapy, i.e. required to achieve (i) immunosuppressive, (ii) antiinflammatory, or (iii) anti-parasitic effectiveness. Thus, in general, satisfactory results are obtained on administration in a dose range of from about 1 or about 5 to about 50 mg/kg/day (e.g., in the case of cyclosporine A, from about 5 or about 10 to about 20 mg/kg/day during the initial phase of therapy, reducing to a maintenance dose of from about 1 or about 5 to about 10 mg/kg/day) administered to the patient orally, once or in divided doses 2 or 3x a day. Where I.V. administration is required, e.g. administration by infusion (for example in the initial phase of treatment) lower dosages, e.g. of the order of from about 0.5 or about 1 to about 10 (e.g. in the case of cyclosporine A, from about 1 or about 3 to about 5 mg/kg/day for an initiating dose, or to about 2.5 mg/kg/day for a maintenance dose) are generally indicated.

The term "antithromboxane $A_2$ agent" is meant to include thromboxane $A_2$ receptor antagonists and thromboxane $A_2$ synthase inhibitors. It is believed that any thromboxane $A_2$ antagonist or thromboxane $A_2$ synthase inhibitor would be useful in the present invention. Non-limiting examples of thromboxane $A_2$ antagonists include:

<u>Antagonists</u>

<u>Compound 1</u>

3-(1-(4-Chlorobenzyl)-3-methyl-5-fluoroindol-2 yl)-2,2-dimethyl propanoic acid, as disclosed in EPO publication 166.591, Example 34.

<u>Compound 2</u>

<u>(RACEMATE)</u>

9-(p-Chlorobenzyl)-6-fluoro-1,2,3,4-tetrahydrocarbazol-1-yl acetic acid, as disclosed in U.S. Patent Application Serial No. 001739, Example 1.

<u>Compound 3</u>

<u>((-) isomer)</u>

(-)-9-(p-Chlorobenzyl)-6,8-difluoro-1,2,3,4-tetrahydrocarbazol-1-yl acetic acid, as disclosed in U.S. Patent Application Serial No. 001739, Example 37.

Compound 4

((-) isomer)

(-)-9-(p-Methylsulfonylbenzyl)-6,8-difluoro-1,2,3,4-tetrahydrocarbazol-1-yl acetic acid, as disclosed in U.S. Patent Application Serial No. 001739, Example 50.

Compound 5

3-(Hydroxymethyl)dibenzo[b,f]thiepin 5,5-dioxide, as disclosed in U.S. Patent 4,237,160, Example 6.

Compound 6

4-(2-((Phenylsulfonyl)amino)ethyl)phenoxyacetic acid, commonly known as BM13177, disclosed in Drugs of the Future, 9, 804-6 (1984).

Compound 7

4-(2-((4-Chlorophenylsulfonyl)amino)ethyl)phenylacetic acid, commonly known as BM 13505, as disclosed in German offen. 2,000, 377.

Compound 8

[1α(Z),2β,5α]-(±)-7-[5-[[(1,1′-Biphenyl)-4-yl] methoxy]-2-(4-morpholinyl)-3-oxocyclopentyl]-4-heptenoic acid, commonly known as AH 23848, as disclosed in Drugs of the Future, 11, 85-8 (1986).

Compound 9

2,2′-Dithiobis(N-(2-hydroxypropyl)benzamide), commonly known as KF-4939, as disclosed in Drugs of the Future, 8, 857 (1983).

Compound 10

[1β,2α (5Z), 3α, 4β]-7-[3-[[2-[(phenylamino) carbonyl]hydrazino]methyl]-7-oxabicyclo[2.2.1]hept 2-yl]-5-heptenoic acid, commonly known as SQ 29548, as disclosed in J. Pharm. Exp. Ther., 234, 435-41 (1985).

Non-limiting examples of thromboxane $A_2$ synthase inhibitors include:

Inhibitors

Compound 11

(E)-2-Methyl-3-(4-(3-pyridinylmethyl)phenyl)-2-propenoic acid, sodium salt, commonly known as OKY-1581, as disclosed in Drugs of the Future, 7, 399 (1982).

Inhibitors continued

Compound 12

(E)-3(4-(1H-Imidazol-1-ylmethyl)phenyl)-2-propenoic acid, monohydrochloride, monohydrate, commonly known as OKY-046, as disclosed in J. Med. Chem., 24, 1139-1148 (1981).

Compound 13

(E)-7-phenyl-7-(3 pyridyl)-6-heptenoic acid, commonly known as CV-4151, as disclosed in Drugs of the Future, 11, 183 (1986).

Compound 14

3-Methyl-2-(3-pyridinyl)-1H-indole-1-octanoic acid, commonly known as CGS-12970, as disclosed in Br. J. Pharmacol. 86, 497-504 (1985).

Compound 15

4-(2-(1H-Imidazol-1-yl)ethoxy)benzoic Acid, monohydrochloride, commonly known as UK-37248 (Dazoxiben), as disclosed in Drugs of the Future, 6, 693-5 (1981).

Inhibitors continued

Compound 16

3-(1H-Imidazol-1-ylmethyl)-2-methyl-1H-indole-1-propanoic acid, commonly known as UK-38485, as disclosed in Drugs of the Future, 8, 947-8 (1983).

The effective daily dosage level for the antithromboxane $A_2$ agent in mammals, especially humans, will generally range from about 0.002 mg/kg to about 100 mg/kg, preferably from about 0.02 mg/kg to about 30 mg/kg. The dosage may be administered in single or divided individual doses.

Any suitable route of administration may be employed for providing a mammal, especially a human, with an effective dosage of the antithromboxane $A_2$ agent. For example, oral, rectal, transdermal, parenteral, intramuscular, intravenous and the like may be employed. Dosage forms include tablets, troches, dispersions, suspensions, solutions, capsules and the like.

The antithromboxane $A_2$ agent can be part of a pharmaceutical composition which contains a pharmaceutically acceptable carrier and optionally other therapeutic ingredients.

The carrier may take a wide variety of forms depending on the form of preparation desired for

administration, e.g., oral or intravenous. In preparing the compositions for oral dosage form, any of the usual pharmaceutical media may be employed, such as, for example, water glycols, oils, alcohols, flavoring agents, preservatives, coloring agents and the like in the case of oral liquid preparations, such as, for example, suspensions, elixirs and solutions; or carriers such as starches, sugars, diluents, granulating agents, lubricants, binders, disintegrating agents and the like in the case of oral solid preparations such as, for example, powders, capsules and tablets. Because of their ease of administration, tablets and capsules represent the most advantageous oral dosage unit form, in which case solid pharmaceutical carriers are obviously employed. If desired, tablets may be sugar coated or enteric coated by standard techniques.

In addition to the common dosage forms set out above, the antithromboxane $A_2$ agent can also be administered by controlled release means and/or delivery devices such as those described in U.S. Patent Nos. 3,845,770; 3,916,899; 3,536,809; 3,598,123; 3,630,200 and 4,008,719, the disclosures of which are hereby incorporated by reference.

The pharmaceutical composition of the present invention suitable for oral administration can be presented as discrete units such as capsules, cachets or tablets each containing a predetermined amount of the active ingredient, as a powder or granules or as a solution or a suspension in an aqueous liquid, a non aqueous liquid, an oil in water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, lubricating, surface active or dispersing agent. Molded tablets may be made by molding in a suitable machine, a mixture of the powdered compound moistened with an inert liquid diluent. Desirably, each tablet contains from about 25 mg to about 500 mg of the active ingredient and each cachet or capsule contains from about 25 to about 500 mg of the active ingredient.

The following examples illustrate the invention.

## EXAMPLE

All experiments were carried out using female spontaneously hypertensive (SHR) rats, weight range 160-200 gm, obtained from Taconic Farms. Animals were dosed for 14 days with either olive oil (0.1 ml/100 gm) or olive oil together with cyclosporine A (Sadimmune oral solution, 100 mg/ml) 25 mg/kg) together with either antagonist vehicle control (either 0.1% $NaHCO_3$, 0.9% saline or 1% Methocel: all at 0.1 ml/100 gm) or a solution or suspension of the thromboxane antagonist in the appropriate vehicle. Four thromboxane antagonists have been studied so far. These were Compounds 1, 2, 3 and 4. Thromboxane antagonists have been administered as the sodium salt dissolved in saline, the sodium salt dissolved in 0.1% $NaHCO_3$ or the free acid suspended in 1% Methocel. The dose of cyclosporine A used produced a significant rise in serum blood urea nitrogen (BUN) after 14 days treatment indicative of abnormal renal function (serum BUN was measured by Lakeshore Diagnostic Laboratories using serum from blood samples obtained from the dorsal aorta 24 hours after the last dose of cyclosporine A). Higher doses of cyclosporine A (50 mg/kg) produced a significant morbidity during the treatment period.

Table 1 shows the effects of Compound 1 administered in 0.1% $NaHCO_3$ on the increase in serum BUN induced by cyclosporine A. At a dose of 10 mg/kg/day significant inhibition (mean of two separate experiments) was seen ($p < 0.05$). No significant inhibition was observed at 3 mg/kg/day. In one experiment, shown in Table 2, significant inhibition of the increase in serum BUN was obtained with the lower dose of Compound 1 (3 mg/kg/day), together with a similar dose of Compound 2. However, when a second experiment was combined together, no significant inhibition was observed, as was the case with the other vehicle.

Table 4 shows the effects of Compound 3, administered as a suspension of the free acid, on cyclosporine A-induced rises in serum BUN and is a combination of two separate experiments. Both doses of Compound 3 (3 and 10 mg/kg/day) produced significant inhibition of the rise in serum BUN. Table 5 shows the effects of Compound 4 administered as a suspension of the free acid on cyclosporine A-induced

rises in serum BUN. As with Compound 3 the compound was effective at both 3 and 10 mg/kg/day. The apparent increase in potency of Compound 3 and 4 as compared to Compound 1 probably reflects the increased intrinsic potency of these compounds for the thromboxane $A_2$ receptor as well as the improved bioavailability and pharmacokinetics of the compounds as compared to Compound 1.

TABLE 1

| Effects of Compound 1 administered in 0.1% $NaHCO_3$ on cyclosporine A-induced nephrotoxicity | | | |
|---|---|---|---|
| Treatment | Serum BUN ± S.E.M. (mg/100ml) | (n)*** | P value compared* to cyclosporine alone |
| Vehicles alone 0.1% $NaHCO_3$ and olive oil | 21.2 ± 1.4 | (5) | <0.01 |
| Cyclosporine A (25 mg/kg/day) | 28.0 ± 1.6 | (9)** | - |
| Cyclosporine A + Compound 1 (3 mg/kg/day) | 28.3 ± 2.2 | (5) | n.s. |
| Cyclosporine A + Compound 1 (10 mg/kg/day) | 23.4 ± 0.9 | (10)** | <0.05 |

* Dunnet's multiple range analysis
** Results obtained from 2 separate experiments
*** n = number of determinations

TABLE 2

| Effects of Compound 1 and Compound 2 in saline on cyclosporine A-induced nephrotoxicity | | | |
|---|---|---|---|
| Experiment 1 | | | |
| Treatment | Serum BUN ± S.E.M. (mg/100 ml) | (n)** | P value compared* to cyclosporine A alone |
| Vehicles alone (saline & olive oil) | 23.6 ± 1.2 | (5) | <0.01 |
| Cyclosporine A 25 mg/kg/day | 30.5 ± 1.0 | (7) | - |
| Cyclosporine A + Compound 1 (3 mg/kg/day) | 24.5 ± 1.1 | (5) | <0.01 |
| Cyclosporine A + Compound 2 (3 mg/kg/day) | 24.6 ± 1.7 | (5) | <0.01 |

* Dunnet's multiple range analysis
** n = number of determinations

TABLE 3

| Effects of Compound 1 and Compound 2 in saline on cyclosporine A-induced nephrotoxicity | | | |
|---|---|---|---|
| Combined experiments | | | |
| Treatment | Serum BUN ± S.E.M. (mg/100 ml) | (n)** | P value compared* to cyclosporine A alone |
| Vehicles alone (saline & olive oil) | 23.6 ± 1.1 | (10) | <0.01 |
| Cyclosporine A 25 mg/kg/day | 30.9 ± 0.8 | (10) | - |
| Cyclosporine A + Compound 1 (3 mg/kg/day) | 28.0 ± 1.6 | (10) | n.s. |
| Cyclosporine A + Compound 2 (3 mg/kg/day) | 30.3 ± 2.0 | (10) | n.s. |

* Dunnet's multiple range analysis
** n = number of determinations

TABLE 4

| Effects of Compound 3 administered as a suspension in 1% Methocel on cyclosporine A-induced nephrotoxicity | | | |
|---|---|---|---|
| Treatment | Serum BUN ± S.E.M. (mg/100 ml) | (n)*** | P value compared to cyclosporine A alone* |
| Vehicles alone (1% Methocel & olive oil) | 22.8 ± 1.1 | (10) | <0.01 |
| Cyclosporine A 25 mg/kg/day | 28.8 ± 0.9 | (10) | - |
| Cyclosporine A + Compound 3 (3 mg/kg/day) | 23.2 ± 0.9 | (10) | <0.01 |
| Cyclosporine A + Compound 3 (10 mg/kg/day) | 24.0 ± 1.1 | (10) | <0.01 |

* Dunnet's multiple range analysis
** Results obtained from 2 separate experiments
*** n = number of determinations

TABLE 5

| Effects of Compound 4 administered as a suspension in 1% Methocel on cyclosporine A-induced nephrotoxicity | | | |
|---|---|---|---|
| Treatment | Serum BUN ± S.E.M. (mg/100 ml) | (n)** | P value compared to cyclosporine A alone* |
| Vehicles alone (1% Methocel & olive oil) | 22.3 ± 0.9 | (8) | <0.01 |
| Cyclosporine A 25 mg/kg/day | 26.4 ± 0.9 | (8) | - |
| Cyclosporine A + Compound 4 (3 mg/kg/day) | 23.5 ± 1.0 | (8) | <0.05 |
| Cyclosporine A + Compound 4 (10 mg/kg/day) | 21.9 ± 0.8 | (8) | <0.01 |

* Kruskal-Wallis multiple comparison test
** n = number of determinations

## Claims

1. The use of an antithromboxane $A_2$ agent for the preparation of a medicament useful for adjunct administration with cyclosporine to limit cyclosporine induced nephrotoxicity.

2. The use in accordance with Claim 1 wherein said antithromboxane $A_2$ agent is selected from the group consisting of:
3-(Hydroxymethyl)dibenzo[b,f]thiepin 5,5-dioxide;
4-(2-((Phenylsulfonyl)amino)ethyl)phenoxyacetic acid;
4-(2-((4-Chlorophenylsulfonyl)amino)ethyl)phenylacetic acid;
[1α(Z),2β,5α]-(±)-7-[5-[[(1,1′-Biphenyl)-4-yl] methoxy]-2-(4-morpholinyl)-3-oxocyclopentyl]-4-heptenoic acid;
2,2′-Dithiobis(N-(2-hydroxypropyl)benzamide), commonly known as KF-4939;
[1β,2α (5Z), 3α, 4β]-7-[3-[[2-[(phenylamino) carbonyl]hydrazino]methyl]-7-oxabicyclo[2.2.1]hept-2- yl]-5-heptenoic acid;
(E)-2-Methyl-3-(4-(3-pyridinylmethyl)phenyl)-2-propenoic acid, sodium salt;
(E)-3(4-(1H-Imidazol-1-ylmethyl)phenyl)-2-propenoic acid, monohydrochloride, monohydrate;
(E)-7-phenyl-7-(3-pyridyl)-6-heptenoic acid;
3-Methyl-2-(3-pyridinyl)-1H-indole-1-octanoic acid;
4-(2-(1H-Imidazol-1-yl)ethoxy)benzoic Acid, monohydrochloride; and
3-(1H-Imidazol-1-ylmethyl)-2-methyl-1H-indole-1-propanoic acid.

3. A pharmaceutical composition which comprises cyclosporine and an antithromboxane $A_2$ agent.